Europäisches Patentamt

**European Patent Office**

**Office européen des brevets**

(19)

(11) Publication number: **0 006 314**
**A1**

# EUROPEAN PATENT APPLICATION

(12)

(21) Application number: **79300950.7**

(22) Date of filing: **25.05.79**

(51) Int. Cl.³: **A 61 F 1/06**

(30) Priority: **31.05.78 GB 2511278**
**07.08.78 US 931319**

(43) Date of publication of application: **09.01.80**
**Bulletin 80/1**

(84) Designated Contracting States: **CH DE FR GB IT NL**

(71) Applicant: **Wadsworth, Thomas Gordon, 22 Hyde Park Square, London, W2 2NL (GB)**

(72) Inventor: **Wadsworth, Thomas Gordon, 22 Hyde Park Square, London, W2 2NL (GB)**

(74) Representative: **King, James Bertram et al, Kings Patent Agency Limited Wardrobe Court 146a Queen Victoria Street, London, EC4V 5AT (GB)**

(54) **Elbow prosthesis and instrumentation for implantation of the prosthesis.**

(57) A prosthesis (2) for total or partial replacement of an elbow comprising:

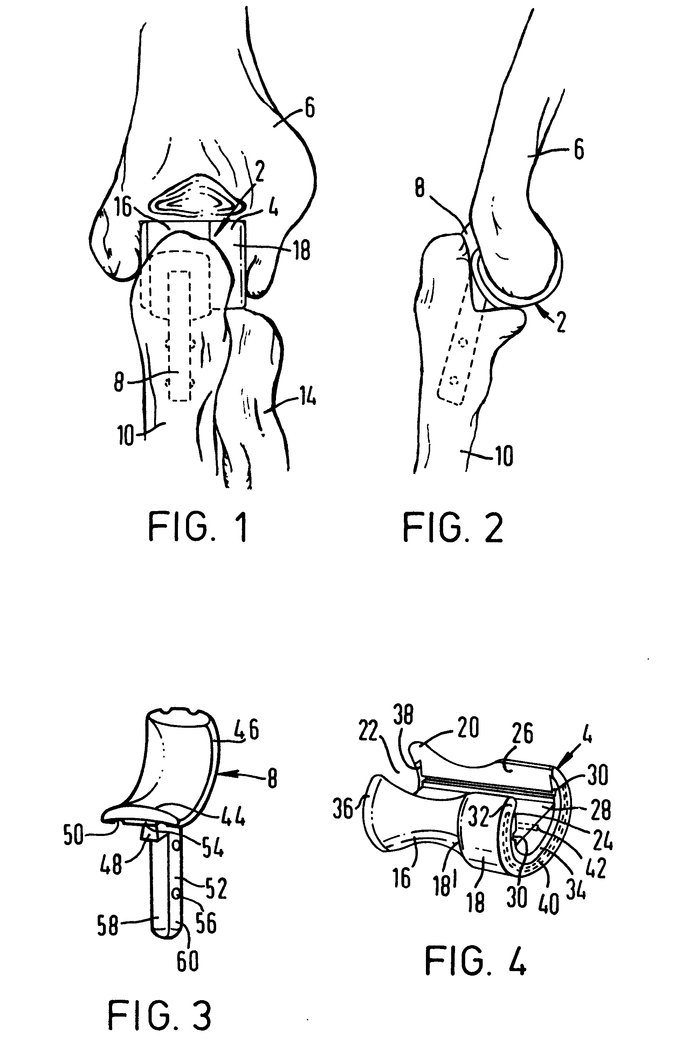

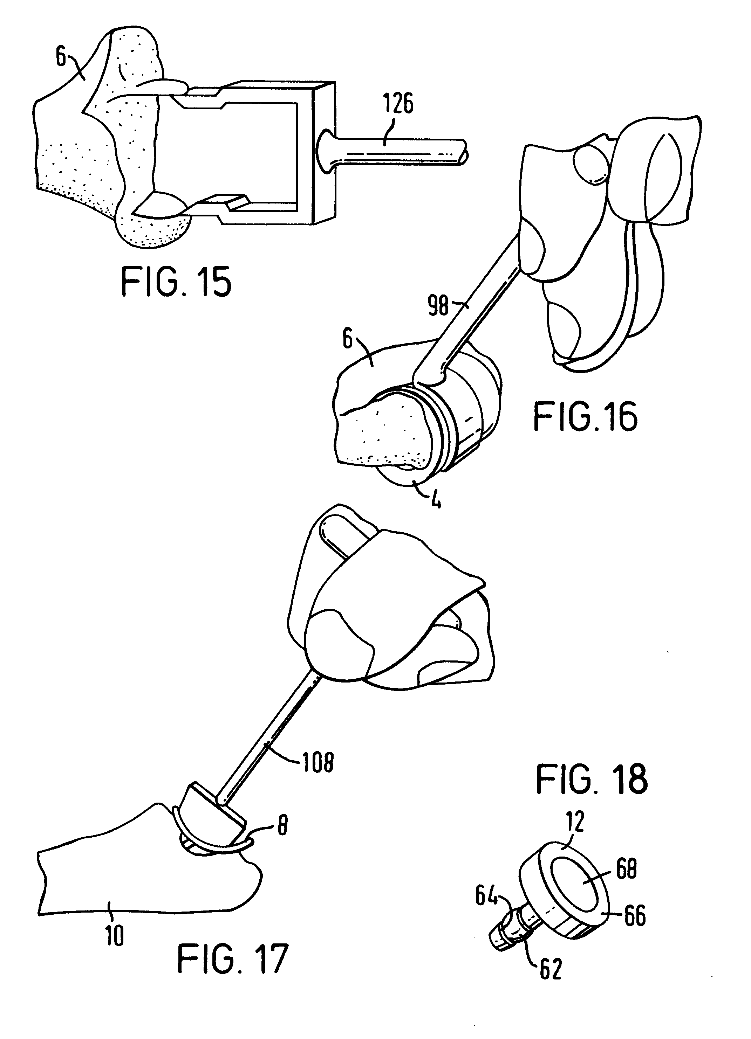

(i) a humeral component (4) to replace the trochlea bone and to partially replace the capitulum bone of the humerus (6), the component (4) being adapted to be fixed to the humerus (6) and having a concave surface (16 – Fig. 4) in the coronal plane, the surface of the component (4) being convex (20 – Fig. 4) in the sagittal plane at the level of the concave surface (16), and (ii) an ulnar component (8) having a corresponding convex surface (44 – Fig. 3) in the coronal plane to articulate with the concave surface (16) of the component (4) and a corresponding concave surface (46 – Fig. 3) in the sagittal plane to articulate with the convex surface (20) of the component (4) in the sagittal plane, any lateral shift of the ulnar component (8) being discouraged in the prosthesis when in situ by the side walls of the concave/convex articulation surface of the component (4). The prosthesis may include (iii) a radial component (12 – Fig. 18).

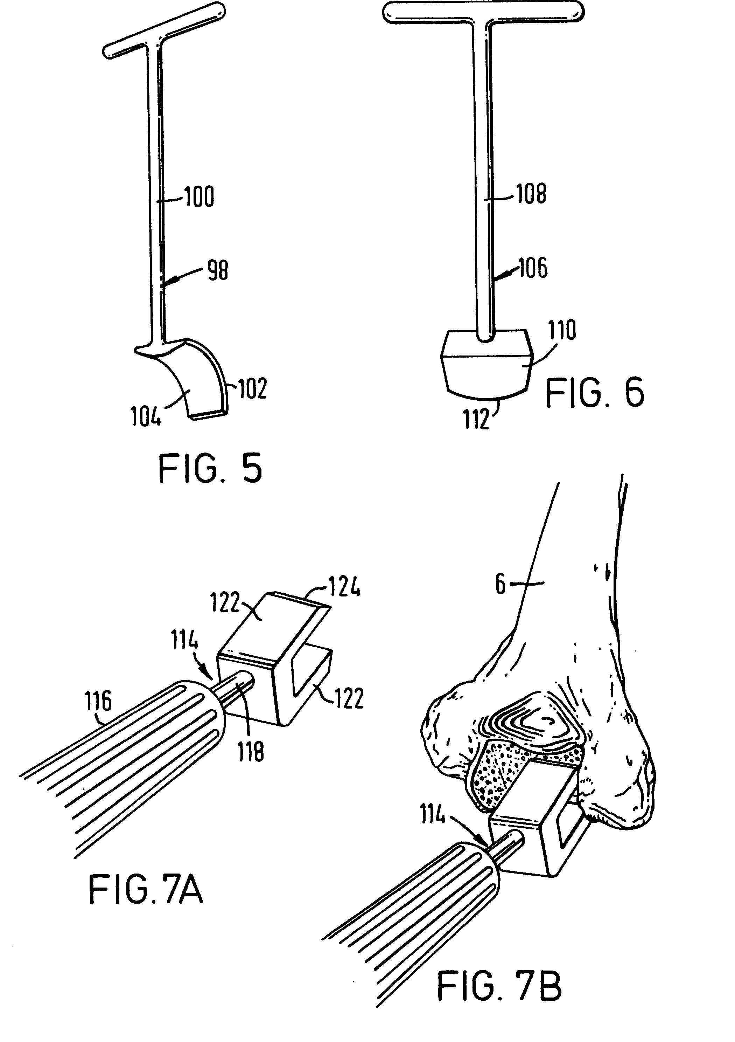

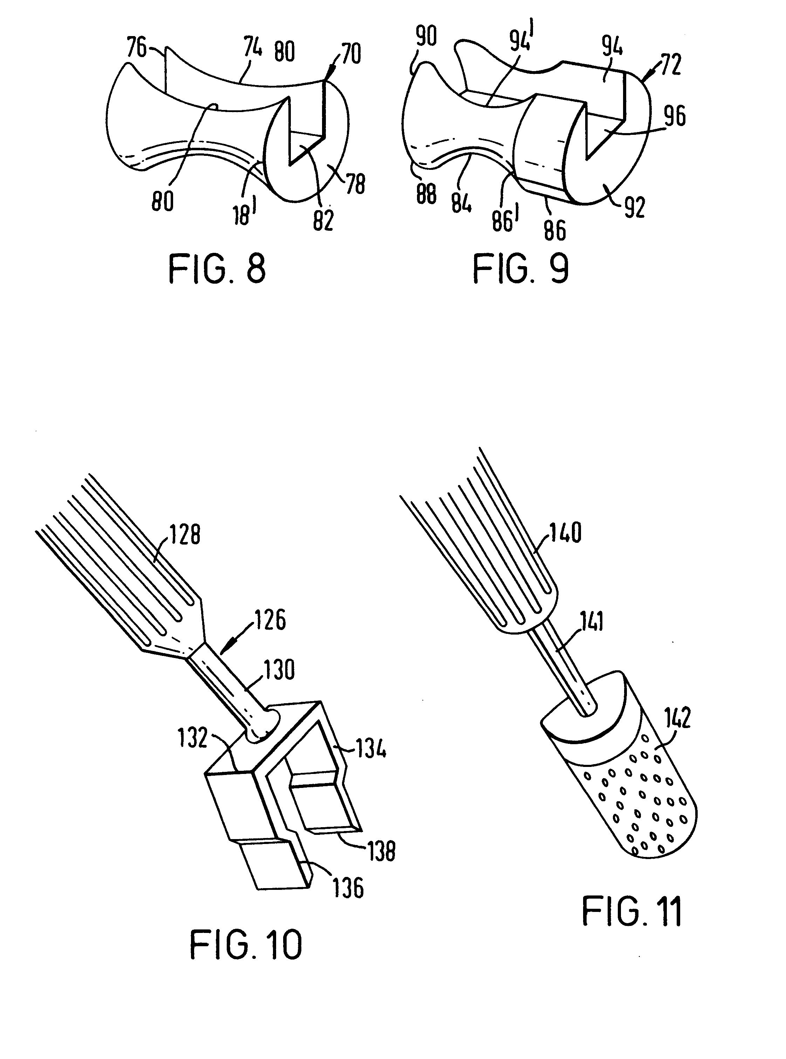

Instrumentation for implantation of the prosthesis comprising a pair of different bone chisels (Figs. 7A and 10), a humeral component impactor (Fig. 5), and ulnar component impactor (Fig. 6) and a rasp (Fig. 11) is also disclosed.

**TITLE MODIFIED   -1-**
**see front page**

"Elbow Replacement Prosthesis"

This invention relates to a prosthesis for total or partial replacement of an eblow, especially arthritic or damaged elbow joints, which is relatively easy to install and which provides a relatively large range of flexible movements.   More particularly, it concerns a form of elbow joint prosthesis that has good joint stability with minimum bone removal while providing normal flexion and extension motion;   there is substantially no in-built constraint and so little strain on the seating of the prosthesis.

In general, prosthetic replacement of the elbow has proved a difficult and often disappointing task over the years and this has been the experience of most surgeons in this field.   In the past, it was common to replace the elbow joint by means of a constrained prosthesis, usually consisting of a large stem inserted into the humerus and another into the ulna and the two parts of the component linked together by an axle pin.   The strong forces on the elbow tend to disrupt the prosthesis from the bone and a very great deal of bone had to be removed to put in such a prosthesis, resulting in the long-term in a disastrous situation very often if the prosthesis has, in fact, to be removed.

There has been a move in recent times to a surface replacement of the elbow joint and my work in this area led to the development of a constrained device with a T-slot stability factor which is described in U.S. Patent Specification 4,079,469. Numerous patent specifications have issued describing bone joint prosthesis of one kind or another but none is wholly satisfactory.

In spite of the numerous procedures and devices previously developed and used for elbow reconstruction, there exists a need for further improvement particularly as regards resulting joint stability, minimal removal of bone for insertion and allowance for normal flexion and extension motion as well as minimal strain on the seating of the prosthesis.

According to the present invention there is provided a prosthesis for total or partial replacement of an eblow comprising a humeral component to replace the trochlea bone and to partially replace the capitulum bone of the humerus, the humeral component being adapted to be fixed to the humerus and having a concave surface in the coronal plane, the surface of the humeral component being convex in the sagittal plane at the level of the concave surface in the coronal plane, and an ulnar component adapted to be

0006314

-3-

seated on and into the olecranon process of the ulnar bone, the ulnar component having a corresponding convex surface in the coronal plane to articulate with the concave surface of the humeral component in the coronal plane and a corresponding concave surface in the sagittal plane to articulate with the convex surface of the humeral component in the sagittal plane, any lateral shift of the ulnar component being discouraged in the prosthesis when in situ by the side walls of the concave/convex articulation surface of the humeral component. Preferably the concave surface of the humeral component ends laterally in a convex boundary surface, the convex boundary surface being in a plane normal to the coronal plane, and generally the humeral component will be one wherein the lateral convex boundary surface is the boundary of a part-cylindrical surface extending in the coronal plane. If desired, the prosthesis may include a radial component. Preferably the radial component comprises a cylindrical head having an end concave surface adapted to articulate with part of the humeral prosthesis.

The humeral component or the ulnar component or each may be made partially of one material and partially of another material. The humeral component

-4-

may be made at least partially of a plastics material,
e.g. high density polyethylene or it may be made at
least partially of a metal or metallic alloy, e.g. an
alloy containing cobalt, chromium and molybdenum.
The ulnar component may be made at least partially
of a metal or metallic alloy, e.g. an alloy containing
cobalt, chromium and molybdenum.   For instance, if
desired, the ulnar component may be made partly of
plastics material, on the surface(s) intended to
articulate with the corresponding surface(s) of the
humeral component (especially when the corresponding
surface(s) of the humeral component is/are made of
metallic alloy or other metal), and partly of metal.
The radial component, when present may be made at
least partially of a metal or metallic material, e.g.
an alloy containing cobalt, chromium and molybdenum.

The invention also provides instrumentation for
implantation of the prosthesis of the invention, the
instrumentation comprising a humeral component impactor
comprising a handle fixed to an end member having an
internal curved pushing surface whose curvature
corresponds to the external concave/convex surface of
the humeral component in the coronal plane, and an
ulnar component impactor comprising a handle fixed to
an end member having an external curved pushing surface whose curvature corresponds to the internal convex/
concave surface of the ulnar component.   Preferably
the instrumentation includes a pair of different bone
chisels, each bone chisel having a head joined to a
base member of inverted U-section by a stem, each limb
of the U-section terminating in a chisel tip, and the
limbs of the U-section of each bone chisel lying in
planes parallel to one another, and at least one of
the bone chisels having its base member of inverted
U-section inclined to its stem.   The instrumentation
may include a rasp or the like to clear the surface
of the olecranon during implantation of the prosethsis.

There is also provided in accordance with the
invention an elbow replacement prosthesis comprising:

A humeral component having an articular surface
that is concave in a coronal plane and convex in a
sagittal plane, and an ulnar component having an
articular surface that is convex in a coronal plane
and concave in a sagittal plane, so as to be
complementary to the articular surface of the
humeral component.

There is further provided instrumentation for
implantation of the prosthesis defined in the preceding
paragraph, comprising a humeral component impactor
comprising a handle fixed to an end member having an impacting surface complementary to the articular
surface of the humeral component, and an ulnar
component impactor comprising a handle fixed to an
end member having an impacting surface complementary
to the articular surface of the ulnar component.

Finally there is provided an elbow replacement
prosthesis comprising an arcuate ulnar component,
said ulnar component having an articular surface and
including, opposite said surface, an arcuate keel
extending therealong and a stem depending from said
keel.

By way of example preferred embodiments of the
present invention will be described with reference to
the accompanying drawings, in which:-

Fig. 1    is a fragmentary posterior elevation of
one form of an elbow prosthesis
embodying the invention illustrated
as implanted in the humerus and ulna,

Fig. 2    is a fragmentary lateral elevation of
the elbow prosthesis of Fig. 1 with
humerus and ulna in extension position (0°),

Fig. 3    is a perpsective view of one form of an
ulnar component of the prosthesis,

Fig. 4    is a perspective view of one form of a
humeral component of the prosthesis,

Fig. 5      is a perspective view of one form of a
            humeral component impactor for implanting
            a prosthesis emboyding the invention,

Fig. 6      is a perspective view of one form of an
            ulnar component impactor for implanting
            a prosthesis embodying the invention,

Fig.7A      is a perspective view of one form of a
            bone chisel used to cut the humerus for
            implanting the humeral component, and
            Fig. 7B is a perpsective view of the
            same bone chisel cutting the humerus for
            implantation of the humeral component,

Fig. 8      is a perspective view of one form of
            a trial humeral component used primarily
            for replacement of a totally destroyed
            elbow joint,

Fig. 9      is a perspective view of another form of
            a trial humeral component which is likely
            to be used more often in practice,

Fig.10      is a perspective view of another form
            of chisel used in implanting the humeral
            component,

Fig.11      is a perspective view of a rasp to clear
            the surface of the olecranon during
            implantation of the prosthesis,

Figs.12A     are side views of a prosthesis
and  12B
             embodying the invention to show range of pivotal movement,

Fig. 13      is a plan view of a more generally preferred form of humeral component of a prosthesis embodying the invention and similar to the humeral component of Fig. 4,

Fig. 14      is a plan view of a special form of humeral component usable for replacement of a totally destroyed elbow joint,

Fig. 15      is a fragmentary perspective view of a form of chisel cutting the humerus for implantation of the humeral component,

Fig. 16      is a fragmentary perspective view of humeral impactor pushing the humeral component onto the humeral bone during cementing,

Fig. 17      is a fragmentary perspective view of an ulnar impactor pushing the ulnar component onto the ulnar bone during cementing,

Fig. 18    is a perspective view of a radial head component that may be used on occasion in an elbow replacement prosthesis embodying the invention,

Fig. 19    is a posterior view of a prothesis embodying the invention,

Fig. 20    is a diagrammatic view showing range of pivotal movement using a prosthesis such as that of Fig. 19,

Fig. 21    is a diagrammatic view of an implanted prosthesis using a prosthesis embodying the invention and which omits any radial component, and

Fig. 22    is similar to that of Fig. 21 but shows a radial component as part of the implanted prosthesis.

Referring in detail to the drawings, an elbow replacement prosthesis 2 basically comprises a humeral component 4 that is implanted in the humerus 6 and an ulnar component 8 implanted in the ulna 10. Optionally, it may comprise a radial component 12 (Fig. 18) that is implanted in the radius 14.

The humeral component 4 has a concave surface 16 in the coronal plane that is joined laterally to a convex boundary surface $18^1$ limiting the part-

cylindrical surface 18, the latter partially replacing the capitulum bone of the humerus upon implantation. In the sagittal plane, the surface 16 becomes convex at 20 at the level of the concave surface in the coronal plane for articulation with the ulnar component 8. The surfaces 16, 18, $18^1$ and 20 form articular surfaces of the humeral component 4.

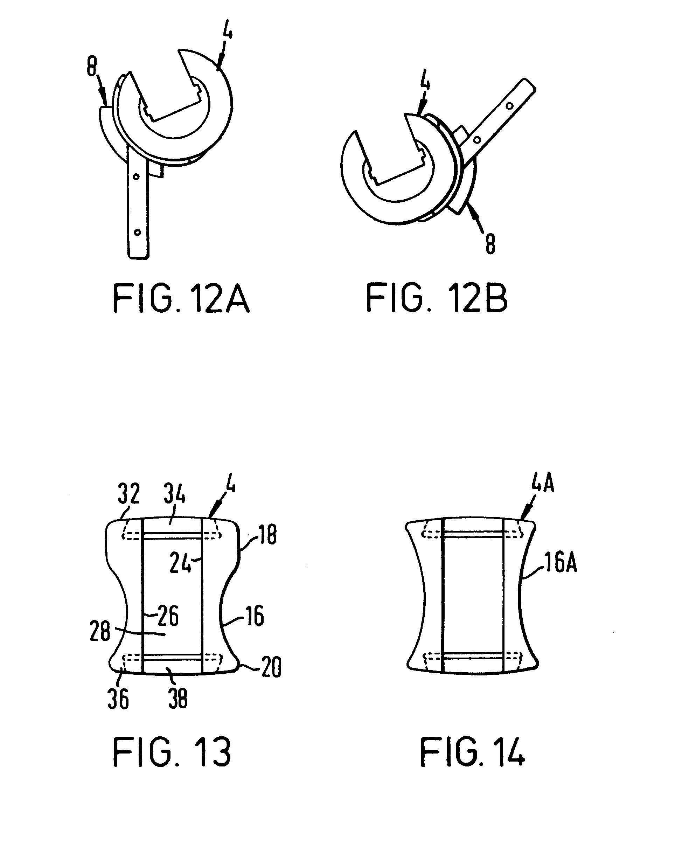

A slightly different form of humeral component 4A is employable in the prosthesis for replacement of a totally destroyed elbow joint (see Figs. 8 and 14). The component 4A is the same in all particulars to component 4 except that a concave articular surface 16A in the coronal plane extends right across the whole length of the component 4A and although there is a convex boundary surface $18^1$ there is no convex surface extending in the coronal plane corresponding to convex surface 18 in component 4 (Fig. 4) or to convex surface 86 in component 70 (Fig. 9).

The surface for attaching the humeral component 4 to the humerus comprises a superior U-slot 22 with an anterior wall 24, posterior wall 26 and flat floor 28. The walls 24 and 26 may be anterior or posterior depending on whether it is the right or left elbow in which the prosthesis is to be inserted - the same prosthesis can be used for right and left.

Longitudinal grooves 30 are formed in the walls 24 and 26 for keying of cement for implantation of the component 4. The lateral wall 32 has a peripheral groove 34 and the medial wall 36 a similar groove 38 for keying of cement. Annular wire markers 40 are embedded in the walls 32 and 36 and a longitudinal wire marker 42 is embedded in the floor 28 so that position of the prosthesis can be determined by X-ray inspection, after implantation, of both the coronal and sagittal planes by means of the three markers.

The main articulating surface of component 4 describes a gentle concave curve in the coronal plane which allows for simple and effective engagement with a convex surface 44 in the coronal plane of the ulnar component 8. Thus, a limited amount of sideways rotation motion is allowed which may at times become necessary in order to avoid undue strain on the bony attachments to the prosthetic components.

In the sagittal plane of the component 4, the concave articulating surface 16 increases in radius from its centre in both the medial and lateral directions until it meets the boundary convex surfaces 18[1] and convex surface 20.

-12-

The articular surface of ulnar component 8 comprises the convex surface 44 in the coronal plane and a concave surface 46 in the sagittal plane. These surfaces allow for accurate and easy articulation with surface 16 of the humeral component 4.

The bone-attaching portion of the ulnar component 8 comprises a longitudinal dove-tailed keel 48 with grooves 50 running longitudinally along beside the keel 48 for keying of cement in implantation. A stem 52 depends from the keel 48 and under-surface 54 of the articulating area for insertion into the ulna. The stem 52 has nipples 56 on both the medial (or lateral) surface 58 and surface 60 for keying into cement for implantation. Surface 60 is lateral if surface 58 is medial or surface 60 is medial if surface 58 is lateral. The length of stem 52 may be increased for special circumstances or other reasons.

The ulnar component 8 is adapted to fit onto and into a surgically prepared ulna. The angle which the stem 52 describes with the articulating area is selected to control anteversion seating of the component 8 in the ulnar bone 10. This is important for antero-posterior stability of the reconstructed elbow joint.

-13-

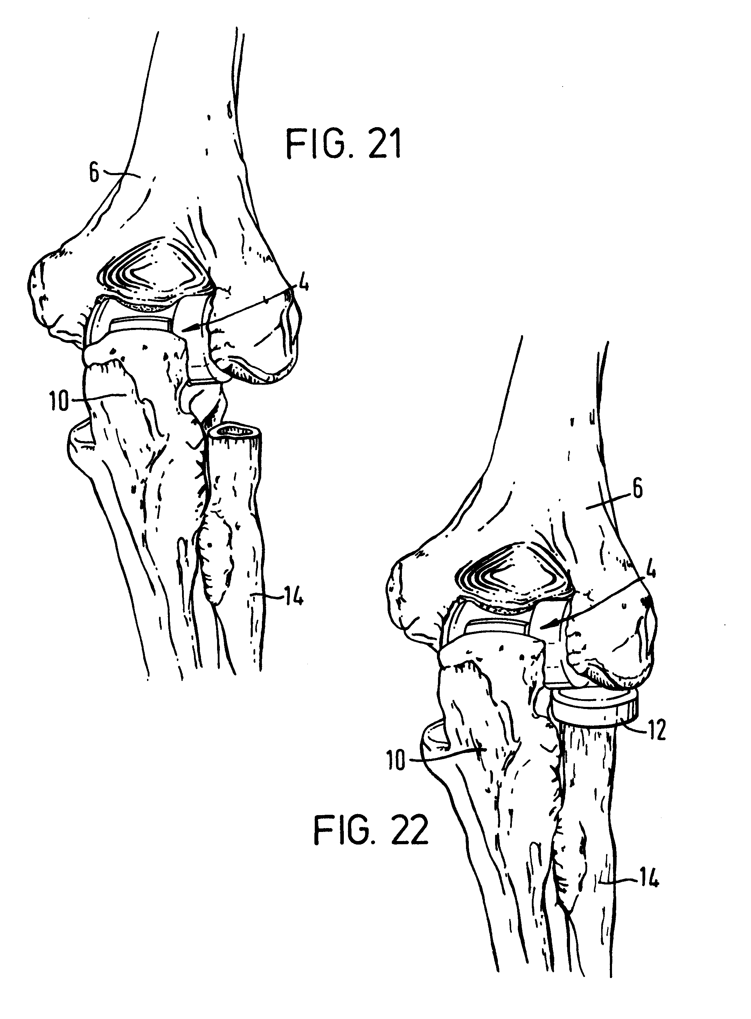

A reconstructed elbow joint employing a
prosthesis embodying the invention may also include
a radial component 12 (Fig. 18).   In actual fact,
although the head of the radius 14 is always
removed for insertion of the prosthesis (Fig. 21),
it is not always necessary to replace this removed
bone.   However, in necessary cases, the bone may be
replaced with a radial component 12 (Figs. 18 and 22)
which comprises a distal key portion 62 having
cement keying grooves 64 and an integral proximal
bearing button 66 with dished surface 68.   If the
radial component 12 is used, it will in practice be
implanted into the surgically prepared radius 14 and
fixed in place with cement in a manner similar to
implant of the ulnar component 8.

In order to assess the depth of humeral bone to
be removed and also for sizing of a component to fit
a particular pateint, trial humeral components may be
used, e.g. humeral component 70 (Fig. 8) or the more
usual humeral component 72 (Fig. 9).   The component
70 corresponds to the component 4A (fig. 14) and
component 72 to the component 4 (Figs. 2, 4 and 13).

The component 70 (Fig. 8) comprises a concave
surface 74 in the coronal plane, lateral wall 76 and
medial wall 78.   Component 70 has U-shaped slot defined by the posterior wall 80, anterior wall $80^1$ and floor 82.

The component 72 (Fig. 9) comprises a concave surface 84 in the coronal plane, convex boundary surface $86^1$ and convex surfaces 86 and 88, medial wall 90, lateral wall 92 and a U-shaped slot defined by posterior wall 94, anterior wall $94^1$ and floor 96.

The components 70 and 72, unlike their counterparts 4 and 4A do not include wire markers or keying grooves, but, aside from this, components 70 and 72 are similar to 4A and 4.

In reconstruction of an elbow joint, only one style of components 4, 4A, 70 or 72 need be used.

A variety of materials is available from which to construct components 4, 8 and 12. Advantageously, the humeral component is formed of inert plastics material, e.g. high density polyethylene, and the ulnar component 8 and radial component 12 are advantageously made of corrosion-resistant metal, e.g. the chromium-containing alloy "Alivium". The impactors and chisels employable for implanting the prosthesis are advantageously formed of stainless steel or equivalent metal or alloy except for the end of the ulnar impactor adapted to make contact with the ulnar preferably metallic prosthesis.

-15-

The design of the components 4 and 8 as described
enables them to be made and used in an average size
adaptable to a large number of patients.   However,
it should be recognized that different sizes may be
necessary and this can be determined by use of
differently dimensioned trial components 70 or 72.

Instrumentation for reconstruction of an elbow
joint is described with reference to Figs. 5 to 7B,
10 and 11.

As seen in Fig. 5 humeral impactor 98 (sometimes
referred to as a pusher) comprises a T-shaped handle
100 adapted to be fixed in the coronal plane to an
integral distal end member 102 having its internal
curved pushing surface 104 formed complementarily to
the concave-convex surface 16 of component 4.   This
enables impactor 98 to be put in position upon the
humeral component easily and be held there accurately
while cement hardens during implantation.

An ulnar impactor 106 (Fig. 6) also has a T-
shaped handle 108, but this is adapted to be fixed in
the sagittal plane to an integral distal end member
110.   The external curved pushing surface 112 is
formed complementarily to the articulating surface
44 of component 8 to again provide easy placement
and accurate holding during cementing.

-16-

Bone chisel 114 (Fig. 7A) comprises a mallet head 116, stem 118, base member 120 and two wings 122, each with chisel tips 124. The stem 118 is fixed to the base member 120, but offset from the centre thereof for ease of access to the elbow joint. Chisel 114 is used to mark out the antero-posterior breadth of the humeral bone to be removed in order to fit into the slot 22 of component 4 (Fig. 4). It also marks the depth equivalent to that of the depth of the slot 22 in component 4.

Bone chisel 126 (Fig. 10) comprises a mallet head 128, stem 130, base member 132, wings 134 with inset ends 136 and chisel tips 138. The wings 134 are in a different plane (relative to stem 130) from the wings 122 of chisel 114 (Fig. 7A).

Chisel 126 (Fig. 10) is used for accuately marking out the length of humeral bone to be removed and also to mark the depth since the inset ends 136 of the chisel are sized in length (i.e. reduced or increased in length) to correspond with the length of the body of component 4. Use of chisel 126 to mark out and cut away humeral bone from the humerus 6 is shown in Fig. 15. Chisel 114 (Fig. 7A) is then used in the opposite plane to mark out the antero-posterior breadth of bone removal. Upon completion of humeral

-17-

bone removal in such manner, the humeral component 4, with cement applied to the surfaces of slot 22 is applied to the humerus 6 and held in place with impactor 98 as shown in Fig. 16 until the cement has set. The keel of the prepared humeral bone sits in the U-slot of component 4 and its medial and lateral ends sit against the medial and lateral walls of the prepared lower humerus.

Fig. 11 shows a rasp with a mallet head 140, stem 141 and base member 142 having a roughened surface and is employable to clear the surface of the olecranon during implantation of the prosthesis.

The ulna is prepared to receive the stem 52 of ulnar component 8 (Fig. 3). Cement is then applied to the stem 52 and under-surface 54 and the component 8 is inserted into and onto the upper end of the ulna 10. The component 8 is held in position as shown in Fig. 17, with impactor 108 until the cement is fully set. In the reconstructed joint, the ulnar component sits on the olecranon process of the ulnar bone and the stem 52 seats within that bone to give good stability to the component 8.

A preferred cement for use in the new joint reconstructions is self-hardening methyl methacrylate cement, but other body-compatible cements may be used.

-18-

As previously indicated, trial humeral components may be used in order to assess the depth of humeral bone to be removed and also for sizing of a component to fit a particular patient.

It is to be understood that Figs. 15 to 17 are illustrative only and do not attempt to depict the actual appearance of the operations. Obviously, the actual surgical procedures would be conducted under sterile conditions with the surgeon's hands gloved and there would be flesh around the humerus and ulna.

In an elbow joint reconstructed by use of a prosthesis embodying a preferred form of the invention, the articulating surfaces, e.g. 16 and 44, fit easily together and generally give a range of flexible movement between full extension ($0^{o}$) to flexion position (approximately $140^{o}$). Stability is provided, in particular, by the intact medial ligament of the elbow and also by the lateral ligament, the anterior and posterior joint capsule and the adjacent musculature. Hence, any tendency for dislocation of the prosthesis is resisted by the natural anatomy and any lateral shift of the ulna is discouraged by the lateral convex boundary surface $18^{1}$ of the humeral component 4. This is useful if trauma or undue strain is delivered to the elbow joint. Further, the prosthetic

-19-

articulating arrangement allows for rotation of the ulna at the elbow, thus avoiding or minising strain on the seating of the two components in strain situations.

Minimal bone is removed in the operative procedure which is a distinct advantage if there should be failure of the prosthesis for any reason, in which event, other surgical procedures can be easily accomplished.

The three humeral wire markers allow for accurate visualisation of the prosthesis by X-ray which is important in assessing wear and/or displacement after accidental trauma or loosening of the humeral component for any reason.

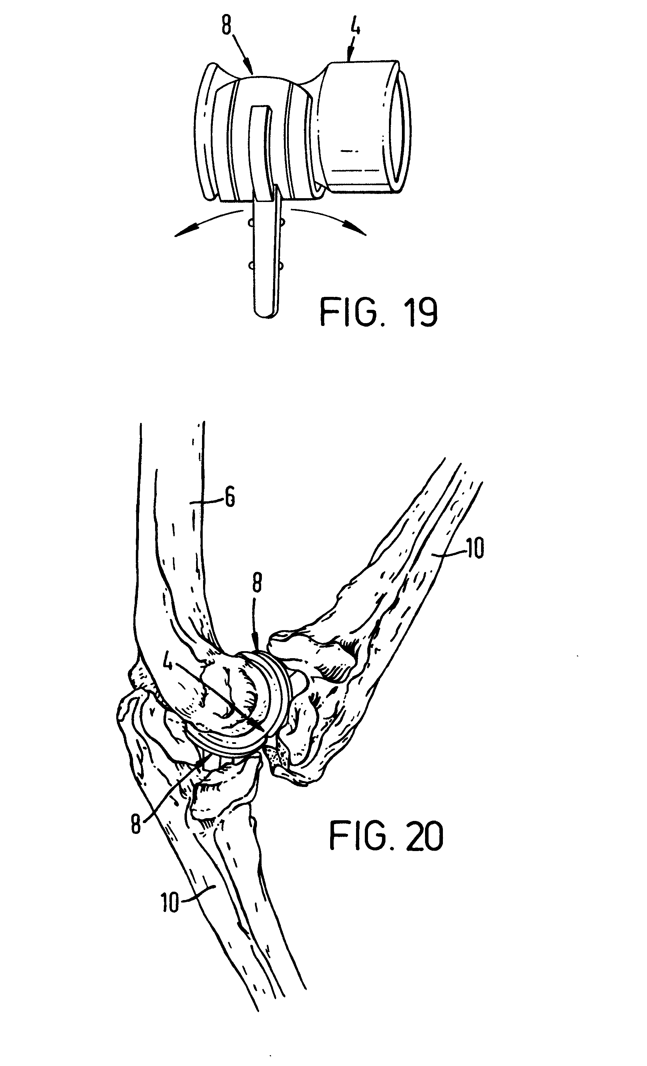

Figs. 12A and 12B show the range of pivotal movement of a prosthesis embodying the invention, Fig. 12A showing one extreme position and Fig. 12B showing the other extreme position of possible pivotal movement. Figs. 19 and 20 also illustrate the pivotal movement (Fig. 20) providing an angular range of $140^{\circ}$ or more and Fig. 19 shows with the two curved arrows pointing in opposite directions the possibility of medio-lateral gliding and limited rotation.

Figs. 21 and 22 are concerned with implanted prosthesis employing prosthesis embodying the

-20-

invention wherein in Fig. 21 a radial component 12 is used fitted to the radius 14 and in Fig. 22 this is omitted, part of the radius bone having been removed by surgery as shown.

-1-

## CLAIMS

1. A prosthesis for total or partial replacement of an elbow comprising a humeral component to replace the trochlea bone and to partially replace the capitulum bone of the humerus, the humeral component being adapted to be fixed to the humerus and having a concave surface in the coronal plane, the surface of the humeral component being convex in the sagittal plane at the level of the concave surface in the coronal plane, and an ulnar component adapted to be seated on the olecranon process of the ulnar bone, the ulnar component having a corresponding convex surface in the coronal plane to articulate with the concave surface of the humeral component in the coronal plane and a corresponding concave surface in the sagittal plane to articulate with the convex surface of the humeral component in the sagittal plane, any lateral shift of the ulnar component being discouraged in the prosthesis when in situ by the side walls of the concave/convex articulation surface of the humeral component.

2. A prosthesis according to Claim 1, wherein the concave surface of the humeral component ends laterally in a convex boundary surface, the convex boundary surface being in a plane normal to the coronal plane.

3. A prosthesis according to Claim 2, wherein the lateral convex boundary surface of the humeral component is the boundary of a part-cylindrical surface extending in the coronal plane.

4. A prothesis according to Claim 1 or Claim 2, which includes a radial component.

5. A prosthesis according to Claim 4, wherein the radial component comprises a cylindrical head having an end concave surface adapted to articulate with part of the humeral prosthesis.

6. A prosthesis according to Claims 3, 4 and 5, wherein the end concave surface is adapted to articulate with the part of the humeral prosthesis replacing the capitulum and with the remainder of the capitulum.

7. A prosthesis according to any preceding claim, wherein the humeral component has a U-slot therein, the U-slot being defined by anterior and posterior walls and a floor.

8. A prosthesis according to Claim 7, wherein there is a longitudinal groove in each of the said walls for keying of cement.

9. A prosthesis according to Claim 7 or Claim 8, wherein indented medial and lateral walls are present and inset into each of these is a wire marker to show the position of the prosthesis on X-ray.

10. A prosthesis according to Claim 9, wherein there is a marker wire extending medially through the body of the humeral component and transversely to the side walls thereof.

11. A prosthesis according to any preceding claim, wherein the humeral component or the ulnar component or each is made partially of one material and partly of another material.

12. A prosthesis according to any preceding claim, wherein the humeral component is made at least partially of a plastics material.

13. A prosthesis according to Claim 12, wherein the plastics material is high density polyethylene.

14. A prosthesis according to any one of Claims 1 to 11, wherein the humeral component is made at least partially of a metal or metallic alloy.

15. A prosthesis according to Claim 14, wherein the metallic material is an alloy containing cobalt, chromium and molybdenum.

16. A prosthesis according to any preceding claim, wherein the surface of the ulnar component for attachment to the ulnar bone comprises a keel of a dove-tailed cross-section and/or longitudinal section.

17. A prosthesis according to Claim 16, wherein on either side of the keel are elongate grooves for keying of cement.

18. A prosthesis according to Claim 16 or Claim 17, which includes a stem extending distally from the keel for insertion into the ulnar bone.

19. A prosthesis according to Claim 18, wherein the stem has nipples on its medial and lateral surfaces for keying of cement.

20. A prosthesis according to any preceding claim, wherein the ulnar component is made at least partially of a metal or metallic alloy.

21. A prosthesis according to Claim 20, wherein the ulnar component is made at least partially of an alloy containing cobalt, chromium and molybdenum.

22. A prosthesis according to Claim 4 or any claim appendant thereto, wherein the radial component has a stem for insertion into the radius.

23. A prosthesis according to Claim 22, wherein the stem has one or more indented rings on it for keying of cement.

-5-

24. A prosthesis according to Claim 4 or any claim appendant thereto, wherein the radial component is made at least partially of a metal or metallic material.

25. A prosthesis according to Claim 24, wherein the radial component is made at least partially of an alloy containing cobalt, chromium and molybdenum.

26. Instrumentation for implantation of the prosthesis claimed in any preceding claim, comprising a humeral component impactor comprising a handle fixed to an end member having an internal curved pushing surface whose curvature corresponds to the external concave/convex surface of the humeral component in the coronal plane, and an ulnar component impactor comprising a handle fixed to an end member having an external curved pushing surface whose curvature corresponds to the internal convex/concave surface of the ulnar component.

27. Instrumentation according to Claim 26, including a pair of different bone chisels, each bone chisel having a head joined to a base member of inverted U-section by a stem, each limb of the U-section terminating in a chisel tip, and the limbs of the U-section of each bone chisel lying in planes parallel to one another, and at least one of the bone chisels having its base member of inverted U-section

0006314

-6-

inclined to its stem.

28. Instrumentation according to Claim 27, wherein the heads of the bone chisels are mallet heads.

29. Instrumentation according to Claim 27 or Claim 28, wherein the ends of the limbs of the U-section of one of the bone chisels are inset with respect to the portions of the limbs merging with the web of the U-section.

30. Instrumentation according to any one of Claims 26 to 28, wherein the handles of the impactors are T-shaped.

31. Instrumentation according to any one of Claims 26 to 30, which includes a rasp or the like to clear the surface of the olecranon during implantation of the prosthesis.

32. An elbow replacement prosthesis comprising:

A humeral component having an articular surface that is concave in a coronal plane and convex in a sagittal plane, and

An ulnar component having an articular surface that is convex in a coronal plane and concave in a sagittal plane, so as to be complementary to the articular surface of the humeral component.

33. An elbow replacement prosthesis comprising an arcuate ulnar component, said ulnar component having an articular surface and including, opposite said surface, an arcuate keel extending therealong and a stern depending from said keel.

34. Instrumentation for implantation of the prosthesis according to Claim 32, comprising a humeral component impactor comprising a handle fixed to an end member having an impacting surface complementary to the articular surface of the humeral component, and an ulnar component impactor comprising a handle fixed to an end member having an impacting surface complementary to the articular surface of the ulnar component.

0006314

FIG. 1

FIG. 2

FIG. 3

FIG. 4

0006314

FIG. 5

FIG. 6

FIG. 7A

FIG. 7B

0006314

FIG. 8

FIG. 9

FIG. 10

FIG. 11

0006314

FIG. 12A

FIG. 12B

FIG. 13

FIG. 14

0006314

FIG. 15

FIG.16

FIG. 18

FIG. 17

0006314

FIG. 19

FIG. 20

0006314

FIG. 21

FIG. 22

## DOCUMENTS CONSIDERED TO BE RELEVANT

| Category | Citation of document with indication, where appropriate, of relevant passages | Relevant to claim |
|---|---|---|
| X | <u>FR - A - 2 300 541</u> (HELFET)<br>* Page 2, line 34 to page 4, line 31; figures *<br><br>-- | 1-6, 11-14, 18-20, 22,24, 32 |
| | <u>GB - A - 1 444 724</u> (HOW MEDICA)<br>* Page 1, line 26 to page 3, line 50; figures *<br><br>-- | 1-4, 12,13, 16,22, 23,32, 33 |
| X | <u>DE - A - 2 404 481</u> (NATIONAL RESEARCH)<br>* Page 2, line 14 to page 10, last line; figures *<br><br>-- | 1-3, 11,13-16,18, 20,21, 32,33 |
| | <u>FR - A - 2 313 906</u> (DOWNS)<br>* Page 1, line 15 to page 4, line 16; figures *<br><br>-- | 1-3, 11,13-15,20, 21,32 |
| X | <u>DE - A - 2 529 238</u> (NATIONAL RESEARCH)<br>* Page 3, line 20 to page 5, line 4; figures *<br><br>-- | 1,7,9-11,13-16,32, 33 |
| | <u>US - A - 3 547 115</u> (STEVENS)<br>* Column 3, line 3 to column 4, line 22; figures *<br><br>-- | 1,2,7-14,27 32 |
| D | <u>US - A - 4 079 469</u> (WADSWORTH)<br>* Column 3, line 51 to column 5, line 12; figures *<br>-- ./. | 1,4-6, 11-15, 18, 20-25 |

### CLASSIFICATION OF THE APPLICATION (Int. Cl.³)

A 61 F 1/06

### TECHNICAL FIELDS SEARCHED (Int.Cl.²)

A 61 F 1/06

### CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 06-09-1979 | LEMERCIER |

EPO Form 1503.1 06.78

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| P | FR - A - 2 403 069 (LEEDS)<br>* Whole *<br>& DE - A - 2 823 406 | 1-7,11,<br>13-25,<br>27-29,<br>32,33 | |
| | ----- | | TECHNICAL FIELDS SEARCHED (Int. Cl.²) |